# EUROPEAN PATENT SPECIFICATION

(11) **EP 0 862 920 B1**
(45) Date of publication and mention of the grant of the patent: **02.01.2003**
(21) Application number: 98100968.1
(22) Date of filing: 21.01.1998
(51) Int. Cl.: A61M 5/32

(54) **Single handedly releasable needle shield**
Einhändig auslösbare Nadelschutzvorrichtung
Dispositif de protection d'aiguille à fermeture déclenchable avec une seule main

(30) Priority: 06.03.1997 US 812747
(43) Date of publication of application: 09.09.1998
(73) Proprietor: Becton Dickinson and Company, Franklin Lakes, New Jersey 07417-1880 (US)
(72) Inventor: Szabo, Sandor, Elmwood Park, N.J. 07407 (US); Odell, Robert B., Franklin Lakes, N.J. 07417 (US)
(74) Representative: Selting, Günther, Dipl.-Ing.

(56) References cited:
- EP-A- 0 724 890
- US-A- 5 445 619
- US-A- 5 599 318
- US-A- 5 603 699

## Description

### BACKGROUND OF THE INVENTION

**1. Field of the Invention.** The subject invention relates to shields for sharply pointed medical implements such as a hypodermic needle, and particularly to shields that can be easily actuated to expose the needle for use.

**2. Description of the Prior Art.** Accidental needle sticks can occur when using medical devices with sharply pointed needles. Accidental needle sticks can be painful and can transmit disease. An accidental stick with a new needle is not likely to transmit disease. The risks associated with accidental needle sticks are well known. Consequently, most prior art needles are provided with safety shields in an effort to reduce the potential for accidental sticks.

Safety shields have taken many different forms. For example, some prior art hypodermic syringes include an elongate rigid cap telescoped over the needle cannula prior to use. Unshielding or reshielding with these prior art caps requires two hands, and therefore is inconvenient. Furthermore reshielding with these prior art caps could be unsafe and is not recommended.

Another prior art safety shield is an elongated rigid tube that is telescoped over the syringe barrel. This prior art safety shield may be releasably retained in a proximal position on the syringe barrel with the needle exposed for use. The rigid tubular safety shield may be advanced distally into a safe shielding position after the needle cannula has been used. Safety shields of this type are very effective, but generally require two-handed actuation.

Some prior art hypodermic syringes include a rigid safety shield hingedly connected to a location near the proximal end of the needle. This prior art safety shield includes an elongate rigid structure having one open side. The open side enables the safety shield to be hingedly rotated from a first position where the needle is substantially surrounded by the safety shield to a second position where the needle is exposed for use. Safety shields of this type are desirable in that a single shield can be used both prior to use and after use of the needle. Safety shields of this type also are desirable in that they may permit single handed unshielding and shielding. However, there is a possibility that the health care worker will touch the tubular side wall of the needle during an unshielding operation. In particular, a health care worker may hold the syringe barrel with the four fingers of one hand while exerting forces on the open side of the shield with the thumb. These forces will cause the shield to hingedly rotate away from the needle cannula. However, the thumb of the health care worker could contact the side of the needle as the safety shield rotates away from the needle. This contact is unlikely to generate an accidental stick with the thumb. However, such contact conceivably could affect the sterility of portions of the needle cannula that may be placed in communication with the patient. Additionally, it is desirable to provide shields that enable a health care worker to keep fingers as far as possible from the needle.

US-A-5,445,619 discloses a shieldable needle assembly corresponding to the pre-characterizing part of Claim 1. This needle assembly has a shield support mounted on a needle hub and carrying a needle shield. This needle shield is an open-sided needle shield which is connected at a proximal end to the shield support. The shield support carries an actuator acting on a toothed gear which is meshing with another toothed gear connected to the needle shield. By rotating the first gear the needle shield is pivoted around its preword axis with respect to the shield support.

### SUMMARY OF THE INVENTION

It is an object of the present invention to provide a shieldable needle assembly of the mentioned type that has an actuator and is simple to manufacture and to use.

The shieldable needle assembly according to the present invention is defined by Claim 1.

The dependent claims define prefered embodiments of the present invention.

The subject invention is directed to a single handedly releasable needle shield for use with a needle cannula. The needle cannula includes a proximal end and a pointed distal end. The proximal end may be securely retained in a needle hub. The needle hub may be releasably securable to the distal end of a prior art syringe. Alternatively, the proximal end of the needle cannula may be permanently secured to the distal end of the syringe.

The safety shield assembly includes an elongate needle shield having opposed proximal and distal ends defining a length greater than the length of the needle cannula. The needle shield includes a rigid substantially U-shaped side wall extending between the proximal and distal ends. The U-shaped side wall is cross-sectionally dimensioned to receive the needle cannula therein. The proximal end of the needle shield is hingedly connected to structure near the proximal end of the needle cannula. For example, the needle shield may be hingedly connected to a needle hub or to the distal end of a syringe barrel. Alternatively, the needle shield may be hingedly mounted to a base which in turn is securely mounted to the needle hub or to the distal end of the syringe barrel. Thus, the needle shield can be hingedly rotated between a shielding position and a ready-to-use position. In the shielding position, the needle cannula is received within the U-shaped side wall of the needle shield. In the ready-to-use position, the needle shield is angularly aligned to and spaced from the needle cannula.

The safety shield assembly of the subject invention further includes a shield actuator near the proximal end of the needle cannula. The shield actuator may be manually deflectable for selectively engaging a portion of the needle shield and for urging the needle shield from the shielding position to the ready-to-use position. For example, the actuator may be disposed to engage portions of the needle shield relatively close to the hinge of the needle shield. At these locations, a relatively small amount of movement generated by the actuator will generate a significant amount of rotational movement.

The needle shield may include at least one releasable locking means for engaging structure on or near the needle cannula when the safety shield is in its shielding position. The releasable locking means functions to prevent unintended rotation of the needle shield from its shielding position to its ready-to-use position. The releasable locking structure is constructed to permit rotation of the needle shield away from the needle cannula in response to digital pressure exerted on the actuator. Digital pressure to unshield the needle cannula must first overcome locking forces between the releasable locking structure and corresponding structure on or near the needle cannula. Once these locking forces have been overcome, however, the digital forces exerted on the actuator will cause the needle shield to rotate about its hinge and toward the ready-to-use position. The hypodermic syringe may then be used in the conventional manner to fill the syringe barrel and/or to inject medication into a patient. The needle cannula may then be shielded by merely exerting digital forces on the needle shield to rotate the needle shield from its ready-to-use position into its shielding position where the needle cannula is enclosed within the U-shaped side wall of the needle shield.

### BRIEF DESCRIPTION OF THE DRAWINGS

Fig. 1 is a perspective view of a shieldable needle assembly in accordance with the subject invention.

Fig. 2 is a partially cross-sectioned side elevational view of the shieldable needle assembly of Fig. 1.

Fig. 3 is a bottom plan view of the shieldable needle assembly of Fig. 1.

Fig. 4 is a cross-sectional view of the shieldable needle assembly of Fig. 2 taken along line 4-4.

Fig. 5 is a perspective view of the shieldable needle assembly illustrating the shield being moved toward the ready-to-use position.

Fig. 6 is a side elevational view of the shieldable needle assembly with the needle cannula exposed.

Fig. 7 is a side elevation view of the distal end of the shieldable needle assembly of Fig. 6.

Fig. 8 is a side elevational view of an alternative embodiment of the shielded needle assembly of the present invention.

### DETAILED DESCRIPTION

While this invention is satisfied by embodiments in many different forms, there are shown in the drawings and will be herein described in detail preferred embodiments of the invention with the understanding that the present disclosure is to be considered exemplary of the principles of the invention and is not intended to limit the scope of the invention to the embodiments illustrated. The scope of the invention will be measured by the appended claims and their equivalents.

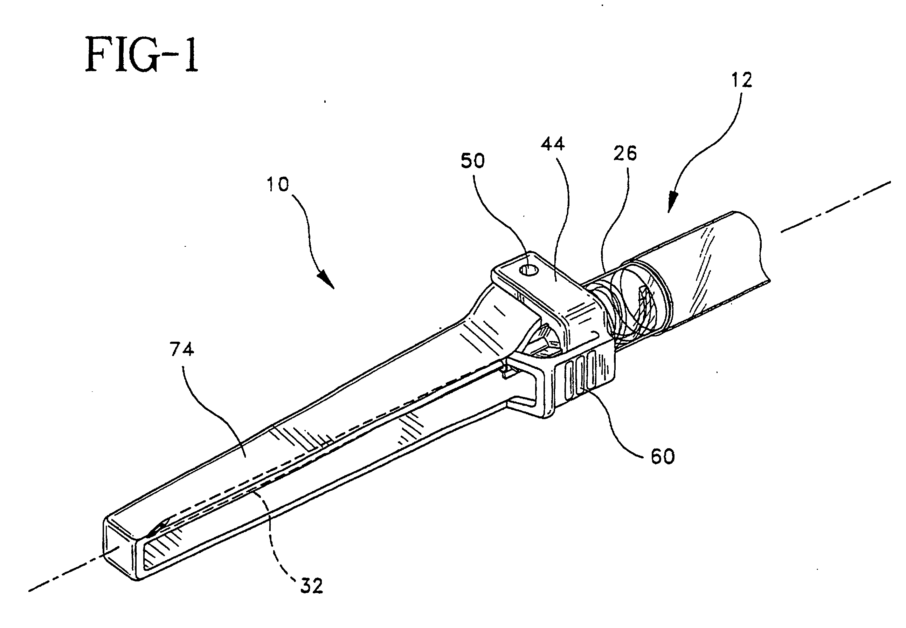

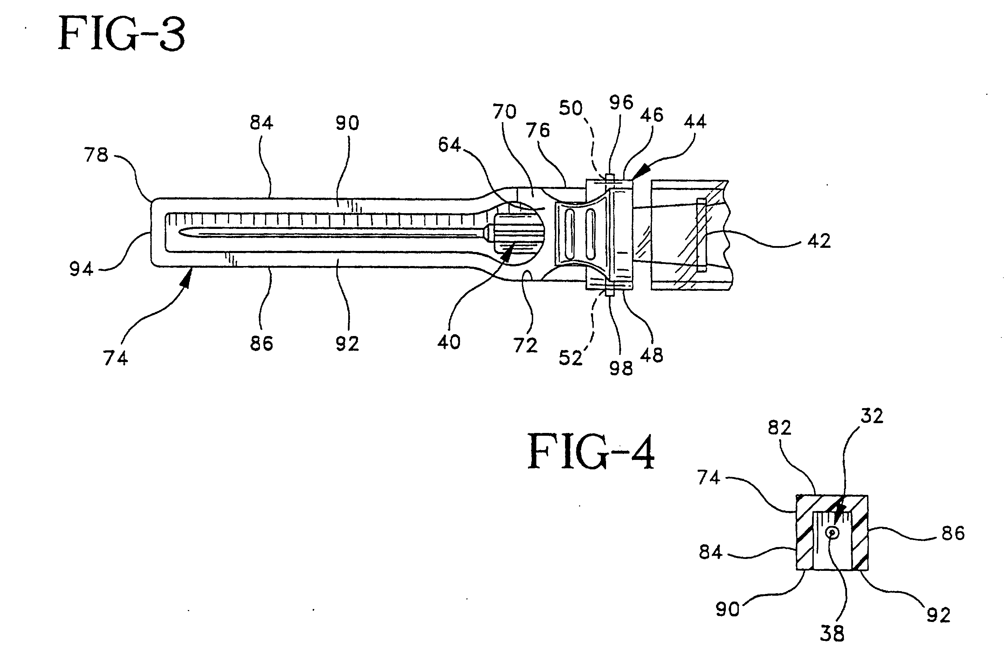

A shieldable needle assembly in accordance with the subject invention is identified generally by the numeral 10 in Figs. 1-7. Shieldable needle assembly 10 is used with a hypodermic syringe 12. Syringe 12 has a barrel 14 with a tubular side wall 16, an open proximal end (not shown), a distal end wall 18 and a fluid receiving chamber 20 extending between the open proximal end and distal end wall 18. A tapered tip 22 projects distally from distal end wall 18 and includes a passage 24 extending therethrough and communicating with chamber 20. A luer collar 26 projects distally from distal end wall 18 of syringe barrel 14 in concentric surrounding relationship to tip 22. Luer collar 26 includes an array of internal threads 28. A plunger 30 extends into the open proximal end of syringe barrel 14 and is in sliding fluid tight engagement with side wall 16. Distal movement of plunger 30 within syringe barrel 14 expels fluid in chamber 20 through passage 24 of tip 22. Proximal movement of plunger 30 within fluid receiving chamber 20 draws fluid through passage 24 of tip 22 and into chamber 20.

Shieldable needle assembly 10 includes a needle cannula 32 having a proximal end 34, an opposed pointed distal end 36 and a lumen 38 extending continuously therebetween. Proximal end 34 of needle cannula 32 is securely embedded in a hub 40. Hub 40 includes luer projections 42 which are releasably threadedly engaged with threads 28 of luer collar 26. Thus, hub 40 enables passage 24 through tip 22 of syringe 12 to be placed in fluid communication with lumen 38 of needle cannula 32.

Hub 40 further includes a shield support 44 at a location spaced sufficiently from luer projections 42 for shield support 44 to preferably be distally of luer collar 26 when hub 40 is threadedly engaged in luer collar 26. As depicted herein, shield support 44 is a unitary part of hub 40. In other embodiments, shield support 44 may be molded separately from hub 40 and may be securely mounted thereon. Shield support 44 includes arms 46 and 48 disposed in spaced parallel relationship to one another on opposite respective sides of needle cannula 32. Arms 46 and 48 include coaxially aligned pivot apertures 50 and 52 respectively which extend substantially orthogonally to needle cannula 32. Additionally, arms 46 and 48 include locking recesses 54 and 56 respectively disposed in opposed facing relationship to one another at locations intermediate pivot aperture 50, 52 and needle cannula 32.

Shieldable needle assembly 10 further includes a shield actuator 60 extending distally from a location on shield support 44 substantially opposite pivot apertures 50 and 52. Thus needle cannula 32 is preferably between actuator 60 and pivot apertures 50, 52. Actuator 60 is unitarily molded with shield support 44, which in turn, is unitarily molded with hub 40. Although not preferred, the actuator, shield support and hub can be unitarily molded with a syringe barrel.

Actuator 60 includes opposed proximal and distal ends 62 and 64 respectively and a trigger 66 therebetween. Proximal end 62 of actuator 60 is unitarily molded with proximal portions of shield support 44. In this embodiment, trigger 66 is substantially planar and extends distally from proximal end 62 of actuator 60 in preferably a substantially parallel relationship to needle cannula 32. Trigger 66 has a plurality of projections 68 for facilitating digital manipulation.

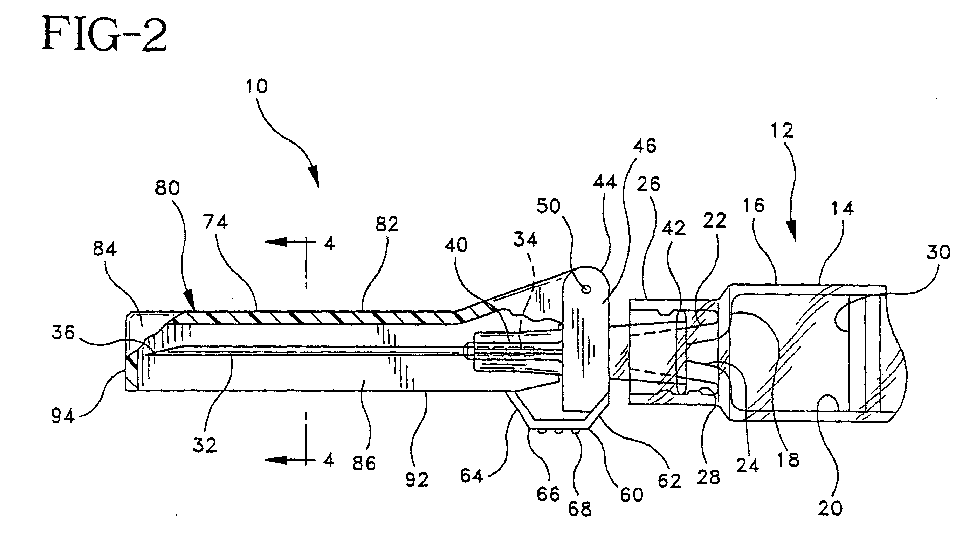

Distal end 64 of actuator 60 is angularly aligned to trigger 66 and projects toward needle cannula 32. Additionally, distal end 64 is preferably U-shaped, and includes side projections 70 and 72. Actuator 60 is formed from a resiliently deflectable plastic material. Thus, digital pressure exerted on trigger 66 will deflect distal end 64 of actuator 60 toward needle cannula 32. Under maximum deflection, side projections 70 and 72 of distal end 64 may pass on opposite respective sides of needle cannula 32. However, upon release of digital pressure from trigger 66, actuator 60 will resiliently return toward an undeflected condition, as shown most clearly in Fig. 2.

Shieldable needle assembly 10 further includes a needle shield 74. Needle shield 74 is an elongate rigid structure preferably unitarily molded from a plastic material and includes a proximal end 76, a distal end 78 and a generally U-shaped enclosure 80 extending continuously therebetween. The U-shaped enclosure 80 is defined by a base wall 82 and opposed side walls 84 and 86 extending substantially parallel to one another from base wall 82. In the condition shown in Fig. 1-4 needle cannula 32 is shielded within base wall 82 and side walls 84 and 86 of the U-shaped enclosure 80. Side walls 84 and 86 terminate at edges 90 and 92 which define an opening to U-shaped enclosure 80 into which needle cannula 32 may be efficiently received. Side walls 84 and 86 are configured such that portions of edges 90 and 92 register with side projections 70 and 72 of actuator 60 as explained further herein. Additionally, side walls 84 and 86 are preferably spaced a distance to be received between arms 46 and 48 of shield support 44. Distal end 78 of needle shield 74 is preferably defined by a distal wall 94 extending between and connecting base wall 82 and side walls 84 and 86 of U-shaped enclosure 80. Distal wall 94, although not necessary, provides rigidity for needle shield 74 and helps prevent contact with pointed distal end 36 of needle cannula 32 when needle shield 74 is in the orientation shown in Figs. 1-4.

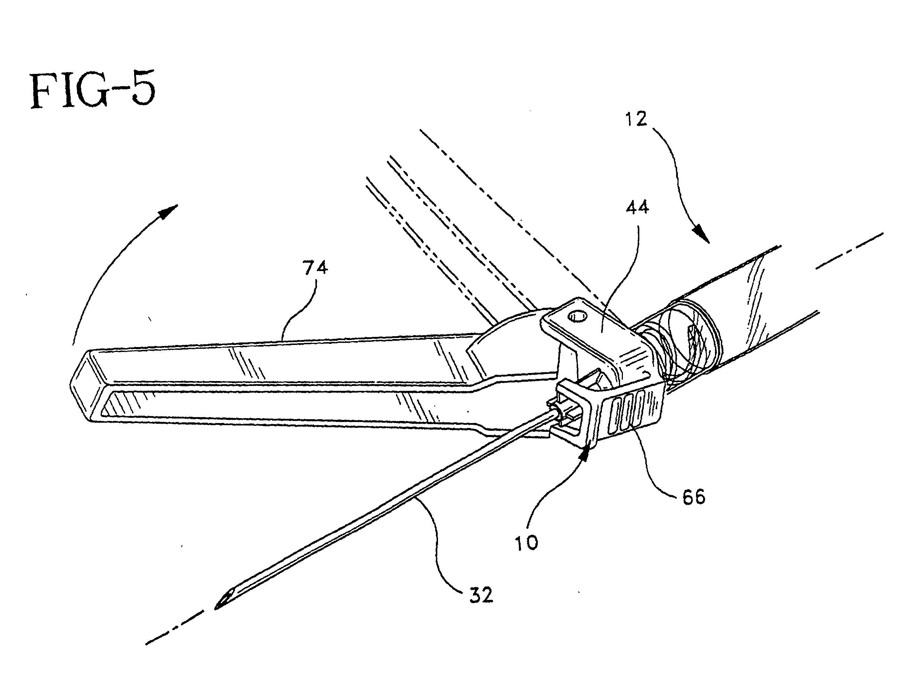

Proximal end 76 of needle shield 74 includes pins 96 and 98 projecting coaxially from side wall 84 and 86 and pivotally received within apertures 50 and 52 of shield support 44. Pins 96 and 98 enable needle shield 74 to be rotated from the shielded orientation shown in Figs. 1-4 to the ready-to-use orientation shown in Figs. 5-7.

Side walls 84 and 86 of needle shield 74 are provided with bosses 100 and 102 substantially adjacent proximal end 76 of needle shield 74. Bosses 100 and 102 are disposed and dimensioned to be snapped into engagement with recesses 54 and 56 in arms 46 and 48 of shield support 44. Side walls 84 and 86 of needle shield 74 are dimensioned to be preferably slidably received between arms 46 and 48 of shield support 44. However, bosses 100 and 102 define a greater dimension than the space between arms 46 and 48 of shield support 44. As a result, arms 46 and 48 of shield support 44 and side walls 84 and 86 of needle shield 74 must be deflected slightly to permit bosses 100 and 102 to be snapped into engagement in recesses 54 and 56 of shield support 44.

Shieldable needle assembly 10 is in a shielding position as shown in Figs. 1-4. In this position needle cannula 32 is received between base wall 82 and side wall 84 and 86 of needle shield 74. Needle shield 74 is releasably locked in this position by engagement of bosses 100 and 102 in recesses 54 and 56 of shield support 44. This structure comprises the preferred means for releasably locking the needle shield in a substantially surrounding disposition relative to the needle. Although two bosses and two recesses are preferred, it is within the purview of the present invention to include only one boss and one recess. Likewise, the positions of the bosses and recesses can be reversed so that the bosses are on the shield support and the recesses are in the needle shield. Many other structures also fall within the purview of the present invention including a projection or projections on the needle shield which engage structure on the needle hub to releasably lock the needle shield. It is also within the purview of the present invention to include releasable locking structure on the needle shield which contacts the needle cannula and deflects the needle cannula or is deflected by the needle cannula as the shield is rotated into the needle protecting position.

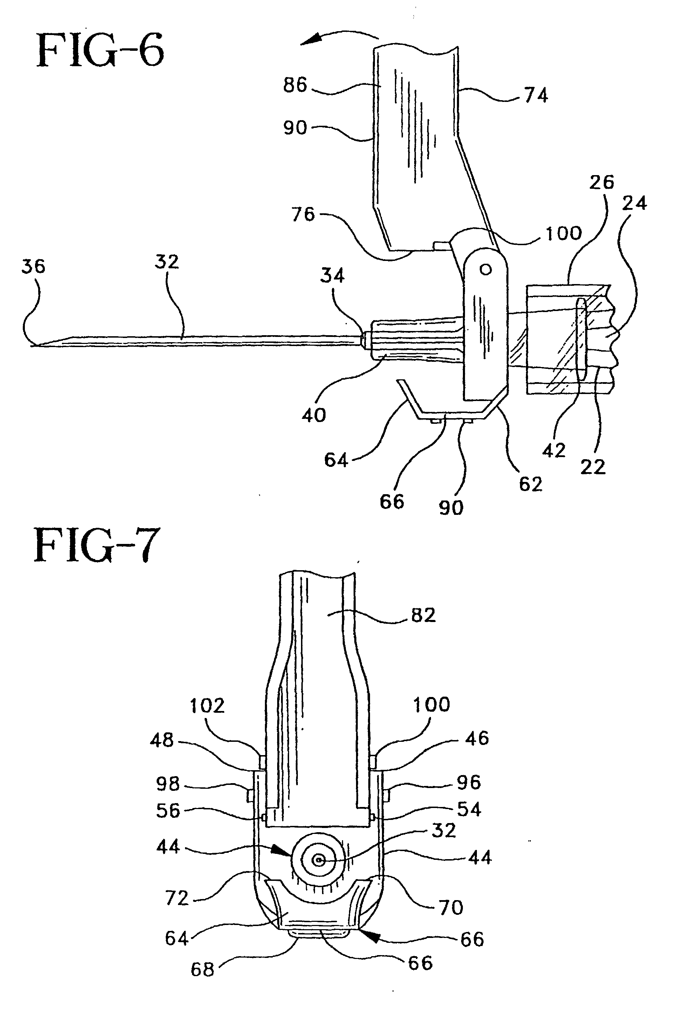

Syringe 12 may be used by exerting digital pressure on trigger 66 of actuator 60. More particularly, a health care worker holding syringe barrel 14 in the forefingers of one hand may exert digital pressure on trigger 68 with the thumb of the same hand. This digital pressure on trigger 66 causes actuator 60 to deflect toward needle cannula 32. Projections 70 and 72 at distal end 64 of actuator 60 will be urged into edges 90 and 92 of side walls 84 and 86 on needle shield 74. These forces will cause bosses 100 and 102 to exert forces on portions of side walls 46 and 48 of shield support 44 adjacent recesses 54 and 56 therein. As a result of these forces, side walls 84 and 86 of needle shield 74 and arms 46 and 48 of shield support 44 will deflect slightly in opposite directions. After sufficient deflection, bosses 100 and 102 will be free of recesses 54 and 56, and the digital forces on trigger 66 of actuator 60 will cause needle shield 74 to accelerate in a pivotal motion about pins 96 and 98 such that needle shield 74 moves into the ready-to-use position shown in Figs. 5 and 6.

Hypodermic syringe 12 may then be used in a conventional manner without interference by needle shield 74. After use, needle cannula 32 may be reshielded by merely exerting digital pressure on base wall 82 of needle shield 74. Such digital pressure will rotate needle shield 74 back toward the shielding condition shown in Figs. 1-4. As needle shield 74 approaches the condition shown in Figs. 1 and 2, bosses 100 and 102 will engage arms 46 and 48 of shield support 44. As noted above, side wall 84 and 86 of shield 74 will undergo a minor deflection in response to these digital forces to permit bosses 100 and 102 to be snapped into engagement with recesses 54 and 56 for releasably locking needle shield 74 in the shielded condition shown most clearly in Figs. 1 and 2.

Both the unshielding and the shielding of needle cannula 32 can be accomplished efficiently by one hand without placing fingers on or near needle cannula 32. In particular, as noted above, unshielding can be achieved by merely exerting digital pressure on actuator 60 at a location proximally of needle cannula 32. Shielding also can be carried out with one hand by merely exerting digital pressure on base wall 82 of needle shield 74 at locations near proximal end of needle shield 74. Movement of bosses 100 and 102 into and out of recesses 54 and 56 in shield support 44 preferably provides both audible and tactile indication that shielding and/or unshielding have been properly completed.

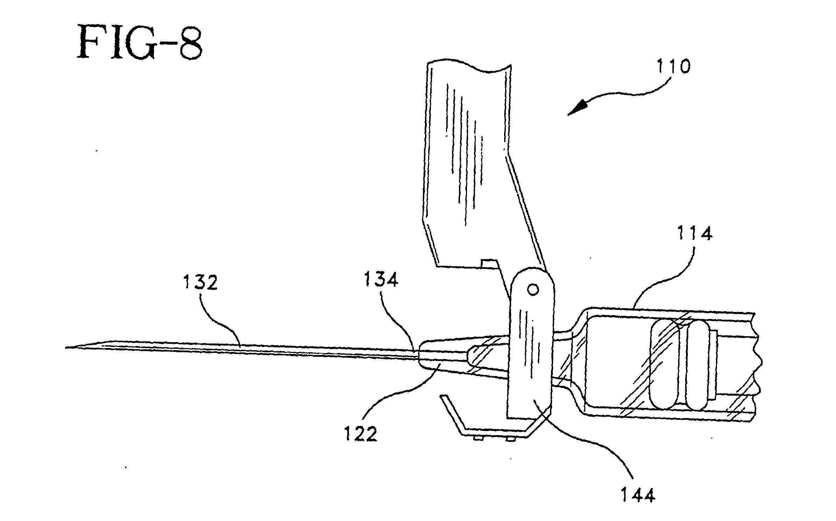

Fig. 8 illustrates an alternative shieldable needle assembly 110 of the present invention. This embodiment functions similarly to the embodiment of Figs. 1-7 except that tip 122 of syringe barrel 114 is integrally formed therewith and functions as the hub does in the embodiments of Figs. 1-7. Proximal end 134 of needle cannula 132 is mounted on tip 122. Likewise, shield support 144 is mounted on tip 122 of the barrel. Accordingly, the tip 122 serves the hub function as if the hub were integrally molded to the syringe barrel. This embodiment of the present invention is ideally suited for prefilled syringes which routinely have needle cannula mounted directly to the syringe barrel without an intermediate hub. Barrel 114 may be made of glass or plastic.

## Claims

1. A shieldable needle assembly comprising:
a needle (32) having a proximal end (34), a distal end (36) and a lumen (38) therethrough;
a needle hub (40) securely mounted to said proximal end of said needle;
a shield support (44) on said needle hub (40);
an open-sided needle shield (74) having a proximal end (76) hingedly connected to said shield support (44), a distal end (78) disposed distally of said needle and a generally U-shaped enclosure (80) extending therebetween and substantially surrounding said needle, said needle shield (74) being hingedly rotatable about said shield support (44) and away from said needle, and
an acuator (60) on said needle shield support (44), for hingedly rotating said needle shield (74) away from said needle,
**characterized in that,**
said actuator (60) is unitary with said shield support (44) and extends from said shield support (44) toward a location on said shield in proximity to said proximal end of said needle, said actuator being deflectable relative to said shield support (44) and said needle (32) for contacting said needle shield (74).

2. The shieldable needle assembly of Claim 1, wherein said shield support (44) and said shield (74) are provided with releasable locking means (54,56;100,102) for releasably locking said needle shield in said substantially surrounding disposition relative to said needle (32).

3. The shieldable needle assembly of Claim 2, wherein said releasable locking structure comprises a boss (100,102) formed on said shield (74) and a boss-receiving recess (54,56) formed on said shield support (44).

4. The shieldable needle assembly of one of Claims 1-3, wherein said actuator (60) projects distally from said shield support (44) and includes projections (70,72) engageable with portions (90,92) of said needle shield on opposite respective sides of said needle.

5. The shieldable needle assembly of one of Claims 1-4, wherein said hub (40) includes projections (42) for releasable threaded engagement of said shieldable needle assembly with a hypodermic syringe (12).

6. The shieldable needle assembly of one of Claims 1-5, wherein said needle shield (74) comprises a base wall (82) and a pair of side walls (84,86) extending from said base wall and disposed on opposite respective sides of said needle, said actuator (60) comprising a pair of projections (70,72) disposed and dimensioned for engaging the respective side walls of said needle shield.

7. The shieldable needle assembly of one of Claims 1-6, wherein said open-sided needle shield (74) is hingedy connected to said shield support (44) at a location spaced to one longitudinal side of said needle, and wherein said actuator is disposed on a longitudinal side of said needle opposite said hinged connection of said needle shield (74) to said shield support (44).

8. The shieldable needle assembly of one of Claims 1-7 connected to a syringe barrel (14) having an elongate cylindrical body defining a chamber (20) for retaining fluid, an open proximal end, a distal end (18) and a tip (22) extending from said distal end, said tip having a tip passageway (24) therethrough in fluid communication with said chamber (20), said tip of said syringe barrel being connected to said hub (40) so that said lumen (38) of said needle is in fluid communication with said chamber (20).

9. The shieldable needle assembly of one of Claims 1-8 wherein said needle hub (122) is integrally formed with a syringe barrel (114) having an elongate cylindrical body defining a chamber for retaining fluid, and open proximal end and a distal end, said needle hub being integrally formed with the distal end of the syringe barrel so that said chamber and said lumen of said needle (132 are in fluid communication.

## Patentansprüche

1. Mit Nadelschutz versehene Nadelvorrichtung mit:
einer Nadel (32) mit einem proximalen Ende (34), einem distalen Ende (36) und einem durch die Nadel verlaufenden Lumen (38);
einem Nadel-Ansatz (40), der fest an dem proximalen Ende der Nadel befestigt ist;
eine Nadelschutz-Halterung (44) an dem Nadel-Ansatz (40);
einer seitlich offenen Nadel-Schutzvorrichtung (74) mit einem schwenkbar an der Nadelschutz-Halterung (44) befestigten proximalen Ende (76), einem distal von der Nadel angeordneten distalen Ende (78) und einer im wesentlichen U-förmigen Einfassung (80), die zwischen den Enden verläuft und die Nadel im wesentlichen umgibt, wobei die Nadel-Schutzvorrichtung (74) gelenkig um die Nadelschutz-Halterung (44) und von der Nadel weg drehbar ist, und
einem an der Nadelschutz-Halterung (44) angeordneten Betätigungsteil (60) zum gelenkigen Drehen der Nadel-Schutzvorrichtung (74) von der Nadel weg,
**dadurch gekennzeichnet, dass**
das Betätigungsteil (60) einstückig mit der Nadelschutz-Halterung (44) ausgebildet ist und sich von der Nadelschutz-Halterung (44) zu einer Stelle an der Schutzvorrichtung erstreckt, die sich nahe an dem proximalen Ende der Nadel befindet, wobei das Betätigungsteil relativ zu der Nadelschutz-Halterung (44) und der Nadel (32) biegbar ist, um die Nadel-Schutzvorrichtung (74) zu kontaktieren.

2. Schützbare Nadelvorrichtung nach Anspruch 1, bei der die Nadelschutz-Halterung (44) und die Schutzvorrichtung (74) mit einer lösbaren Verriegelungseinrichtung (54,56;100,102) versehen sind, um die Nadel-Schutzvorrichtung in der die Nadel (32) im wesentlichem umgebenen Position lösbar zu verriegeln.

3. Schützbare Nadelvorrichtung nach Anspruch 2, bei der die lösbare Verriegelungsstruktur einen an der Schutzvorrichtung (74) ausgebildeten Vorsprung (100,102) und eine an der Nadelschutz-Halterung (44) ausgebildete Vertiefung (54,56) zur Aufnahme des Vorsprungs aufweist.

4. Schützbare Nadelvorrichtung nach einem der Ansprüche 1-3, bei der das Betätigungsteil (60) distal von der Nadelschutz-Halterung (44) absteht und Vorsprünge (70,72) aufweist, die mit Teilen (90,92) der Nadel-Schutzvorrichtung zusammengreifen können, welche an jeweiligen einander gegenüberliegenden Seiten der Nadel angeordnet sind.

5. Schützbare Nadelvorrichtung nach einem der Ansprüche 1-4, bei der der Ansatz (40) Vorsprünge (42) zum lösbaren Gewindeeingriff der schützbaren Nadelvorrichtung mit einer Injektionsspritze (12) aufweist.

6. Schützbare Nadelvorrichtung nach einem der Ansprüche 1-5, bei der die Nadel-Schutzvorrichtung (74) eine Basiswand (82) und ein Paar von Seitenwänden (84,86) aufweist, die von der Basiswand abstehen und an jeweiligen einander gegenüberliegenden Seiten der Nadel angeordnet sind, wobei das Betätigungsteil (60) ein Paar von Vorsprüngen (70,72) aufweist, die zum Angreifen an den jeweiligen Seitenwänden der Nadel-Schutzvorrichtung angeordnet und dimensioniert sind.

7. Schützbare Nadelvorrichtung nach einem der Ansprüche 1-6, bei der die mit offener Seite ausgebildete Nadel-Schutzvorrichtung (74) an einer im Abstand zu einer Längsseite der Nadel gelegenen Stelle gelenkig mit der Nadelschutz-Halterung (44) verbunden ist, und bei der das Betätigungsteil an einer Längsseite der Nadel gegenüber der Schwenkverbindung der Nadel-Schutzvorrichtung (74) und der Nadelschutz-Halterung (44) angeordnet ist.

8. Schützbare Nadelvorrichtung nach einem der Ansprüche 1-7, verbunden mit einem Spritzenzylinder (14), der einen länglichen Zylinderkörper, welcher eine Kammer (20) zur Aufnahme von Fluid bildet, ein offenes proximales Ende, ein distales Ende (18) und einen von dem distalen Ende abstehenden Stutzen (22) mit einem durch diesen verlaufenden Stutzen-Durchlass (24) aufweist, der in Fluidverbindung mit der Kammer (20) steht, wobei der Stutzen des Spritzenzylinders derart mit dem Ansatz (40) verbunden ist, dass sich das Lumen (38) der Nadel in Fluidverbindung mit der Kammer (20) befindet.

9. Schützbare Nadelvorrichtung nach einem der Ansprüche 1-8, bei der der Nadel-Ansatz (122) einstückig mit einem Spritzenzylinder (114) verbunden ist, der einen länglichen Zylinderkörper, welcher eine Kammer zur Aufnahme von Fluid bildet, und ein offenes proximales Ende und ein distales Ende hat, wobei der Nadel-Ansatz einstückig derart mit dem distalen Ende des Spritzenzylinders verbunden ist, dass sich die Kammer und das Lumen der Nadel (132) in gegenseitiger Fluidverbindung befinden.

## Revendications

1. Ensemble d'aiguille pouvant être protégée comportant :
une aiguille (32) ayant une extrémité proximale (34), une extrémité distale (36) et un passage traversant (38),
un moyeu d'aiguille (40) monté de manière ferme sur ladite extrémité proximale de ladite aiguille,
un support de protection (44) situé sur ledit moyeu d'aiguille (40),
une protection d'aiguille ayant un côté ouvert (74) ayant une extrémité proximale (76) reliée de manière articulée audit support de protection (44), une extrémité distale (78) disposée distalement par rapport à ladite aiguille et une enceinte de manière générale en forme de U (80) s'étendant entre celles-ci et entourant pratiquement ladite aiguille, ladite protection d'aiguille (74) pouvant tourner de manière articulée autour dudit support de protection (44) et en s'éloignant à partir de ladite aiguille, et
un actionneur (60) situé sur ledit support de protection d'aiguille (44), pour faire tourner de manière articulée ladite protection d'aiguille (74) loin de ladite aiguille,
**caractérisé en ce que**,
ledit actionneur (60) est en un seul bloc avec ledit support de protection (44) et s'étend à partir dudit support de protection (44) en direction d'un emplacement situé sur ladite protection à proximité de ladite extrémité proximale de ladite aiguille, ledit actionneur pouvant être déformé par rapport audit support d'aiguille (44) et ladite aiguille (32) pour venir en contact avec ladite protection d'aiguille (74).

2. Ensemble d'aiguille pouvant être protégée selon la revendication 1, dans lequel ledit support de protection (44) et ladite protection (74) sont munis de moyens de blocage libérables (54, 56, 100, 102) pour bloquer de manière libérable ladite protection d'aiguille dans ladite disposition entourant sensiblement par rapport à ladite aiguille (32).

3. Ensemble d'aiguille pouvant être protégée selon la revendication 2, dans lequel ladite structure de blocage libérable comporte un bossage (100, 102) formé sur ladite protection (74) et un évidement de réception de bossage (54, 56) formé sur ledit support de protection (44).

4. Ensemble d'aiguille pouvant être protégée selon l'une quelconque des revendications 1 à 3, dans lequel ledit actionneur (60) fait saillie distalement à partir dudit support de protection (44) et comporte des saillies (70, 72) pouvant coopérer avec des parties (90, 92) de ladite protection d'aiguille situées sur des côtés respectifs opposés de ladite aiguille.

5. Ensemble d'aiguille pouvant être protégée selon l'une quelconque des revendications 1 à 4, dans lequel ledit moyeu (40) comporte des saillies (42) destinées à la mise en prise vissée libérable dudit ensemble d'aiguille pouvant être protégée avec une seringue hypodermique (12).

6. Ensemble d'aiguille pouvant être protégée selon l'une quelconque des revendications 1 à 5, dans lequel ladite protection d'aiguille (74) comporte une paroi de base (82) et une paire de parois latérales (84, 86) s'étendant à partir de ladite paroi de base et disposées sur des côtés respectifs opposés de ladite aiguille, ledit actionneur (60) comportant une paire de saillies (70, 72) disposées et dimensionnées pour venir en contact avec les parois latérales respectives de ladite protection d'aiguille.

7. Ensemble d'aiguille pouvant être protégée selon l'une quelconque des revendications 1 à 6, dans lequel ladite protection d'aiguille ayant un côté ouvert (74) est reliée de manière articulée audit support de protection (44) en un emplacement espacé par rapport à un côté longitudinal de ladite aiguille, et dans lequel ledit actionneur est disposé sur un côté longitudinal de ladite aiguille opposé à ladite liaison articulée de ladite protection d'aiguille (74) sur ledit support de protection (44).

8. Ensemble d'aiguille pouvant être protégée selon l'une quelconque des revendications 1 à 7, relié à une partie cylindrique de seringue (14) ayant un corps cylindrique allongé définissant une chambre (20) pour retenir un fluide, une extrémité proximale ouverte, une extrémité distale (18) et un embout (22) s'étendant à partir de ladite extrémité distale, ledit embout ayant un passage d'embout (24) traversant en communication de fluide avec ladite chambre (20), ledit embout de ladite partie cylindrique de seringue étant relié audit moyeu (40) de sorte que ledit passage (38) de ladite aiguille est en communication de fluide avec ladite chambre (20).

9. Ensemble d'aiguille pouvant être protégée selon l'une quelconque des revendications 1 à 8, dans lequel ledit moyeu d'aiguille (122) est formé en un seul bloc avec une partie cylindrique de seringue (114) ayant un corps cylindrique allongé définissant une chambre de retenue de fluide, et une extrémité proximale ouverte et une extrémité distale, ledit moyeu d'aiguille étant formé en un seul bloc avec l'extrémité distale de la partie cylindrique de seringue de sorte que ladite chambre et ledit passage de ladite aiguille (132) sont en communication de fluide.
